# EUROPEAN PATENT APPLICATION

(11) **EP 1 352 963 A1**
(43) Date of publication of application: **15.10.2003**
(21) Application number: 03015327.4
(22) Date of filing: 20.05.1998
(51) Int. Cl.: C12N 15/63, C12N 15/82

(54) **Gene silencing**

(30) Priority: 21.05.1997 GB 9710475
(62) Divisional of application: 98921662.7
(71) Applicant: Syngenta Limited, Guildford Surrey GU2 7YH (GB)
(72) Inventor: Grierson, Donald, Loughborough, LE12 5RD (GB); Lowe, Alexandra Louise, Loughborough, LE12 5RD (GB); Hamilton, Andrew John, Loughborough, LE12 5RD (GB)
(74) Representative: Huskisson, Frank Mackie

(57) **Abstract**

Constructs and methods for enhancing the inhibition of a target gene within an organism involve inserting into the gene silencing vector an inverted repeat sequence of all or part of a polynucleotide region within the vector. The inverted repeat sequence may be a synthetic polynucleotide sequence or comprise a modified natural polynucleotide sequence.

## Description

This invention relates to the control of gene expression, more particularly to the inhibition of expression, commonly referred to as "gene silencing".

Two principal methods for the modulation of gene expression are known. These are referred to in the art as "antisense downregulation" and "sense downregulation"(also, referred to as "cosuppression"). Both of these methods lead to an inhibition of expression of the target gene.

In antisense downregulation, a DNA which is complementary to all or part of an endogenous target gene is inserted into the genome in reverse orientation. While the mechanism has not been fully elucidated, one theory is that transcription of such an antisense gene produces mRNA which is complementary in sequence to the mRNA product transcribed from the endogenous gene: that antisense mRNA then binds with the naturally produced "sense" mRNA to form a duplex which inhibits translation of the natural mRNA to protein. It is not necessary that the inserted antisense gene be equal in length to the endogenous gene sequence: a fragment is sufficient. The size of the fragment does not appear to be particularly important. Fragments as small as 42 or so nucleotides have been reported to be effective. Generally somewhere in the region of 50 nucleotides is accepted as sufficient to obtain the inhibitory effect. However, it has to be said that fewer nucleotides may very well work: a greater number, up to the equivalent of full length, will certainly work. It is usual simply to use a fragment length for which there is a convenient restriction enzyme cleavage site somewhere downstream of fifty nucleotides. The fact that only a fragment of the gene is required means that not all of the gene need be sequenced. It also means that commonly a cDNA will suffice, obviating the need to isolate the full genomic sequence.

The antisense fragment does not have to be precisely the same as the endogenous complementary strand of the target gene. There simply has to be sufficient sequence similarity to achieve inhibition of the target gene. This is an important feature of antisense technology as it permits the use of a sequence which has been derived from one plant species to be effective in another and obviates the need to construct antisense vectors for each individual species of interest. Although sequences isolated from one species may be effective in another, it is not infrequent to find exceptions where the degree of sequence similarity between one species and the other is insufficient for the effect to be obtained. In such cases, it may be necessary to isolate the species-specific homologue.

Antisense downregulation technology is well-established in the art. It is the subject of several textbooks and many hundreds of journal publications. The principal patent reference is European Patent No. 240,208 in the name of Calgene Inc. There is no reason to doubt the operability of antisense technology. It is well-established, used routinely in laboratories around the world and products in which it is used are on the market.

Both overexpression and downregulation are achieved by "sense" technology. If a full length copy of the target gene is inserted into the genome then a range of phenotypes is obtained, some overexpressing the target gene, some underexpressing. A population of plants produced by this method may then be screened and individual phenotypes isolated. A similarity with antisense is that the inserted sequence need not be a full length copy. The principal patent reference on cosuppression is European Patent 465,572 in the name of DNA Plant Technology Inc. There is no reason to doubt the operability of sense/cosuppression technology. It is well- established, used routinely in laboratories around the world and products in which it is used are on the market.

Sense and antisense gene regulation is reviewed by Bird and Ray in Biotechnology and Genetic Engineering Reviews 9: 207-227 (1991). The use of these techniques to control selected genes in tomato has been described by Gray et.al., Plant Molecular Biology, 19: 69-87 (1992).

Gene silencing can therefore be achieved by inserting into the genome of a target organism an extra copy of the target gene coding sequence which may comprise either the whole or part or be a truncated sequence and may be in sense or antisense orientation. Additionally, intron sequences which are obtainable from the genomic gene sequence may be used in the construction of suppression vectors. There have also been reports of gene silencing being achieved within organisms of both the transgene and the endogenous gene where the only sequence identity is within the promoter regions.

Gene control by any of the methods described requires insertion of the sense or antisense sequence, under control of appropriate promoters and termination sequences containing polyadenylation signals, into the genome of the target plant species by transformation, followed by regeneration of the transformants into whole plants. It is probably fair to say that transformation methods exist for most plant species or can be obtained by adaptation of available methods.

The most widely used method is *Agrobacterium-* mediated transformation, mainly for dicotyledonous species. This is the best known, most widely studied and, therefore, best understood of all transformation methods. The rhizobacterium *Agrobacterium tumefaciens,* or the related *Agrobacterium rhizogenes,* contain certain plasmids which, in nature, cause the formation of disease symptoms, crown gall or hairy root tumours, in plants which are infected by the bacterium. Part of the mechanism employed by *Agrobacterium* in pathogenesis is that a section of plasmid DNA which is bounded by right and left border regions is transferred stably into the genome of the infected plant. Therefore, if foreign DNA is inserted into the so-called "transfer" region (T-region) in substitution for the genes normally present therein, that foreign gene will be transferred into the plant genome. There are many hundreds of references in the journal literature, in textbooks and in patents and the methodology is well-established.

Various methods for the direct insertion of DNA into the nucleus of monocot cells are known.

In the ballistic method, microparticles of dense material, usually gold or tungsten, are fired at high velocity at the target cells where they penetrate the cells, opening an aperture in the cell wall through which DNA may enter. The DNA may be coated on to the microparticles or may be added to the culture medium.

In microinjection, the DNA is inserted by injection into individual cells via an ultrafine hollow needle.

Another method, applicable to both monocots and dicots, involves creating a suspension of the target cells in a liquid, adding microscopic needle-like material, such as silicon carbide or silicon nitride "whiskers", and agitating so that the cells and whiskers collide and DNA present in the liquid enters the cell.

In summary, then, the requirements for gene silencing using both sense and antisense technology are known and the methods by which the required sequences may be introduced are known.

The present invention aims to, *inter alia,* provide a method of enhancing the control of gene expression.

According to the present invention there is provided a vector for enhancing the inhibition of a selected target gene within an organism, comprising a gene silencing vector characterised in that the said gene silencing vector includes a inverted repeat of all or part of a polynucleotide region within the vector.

The inverted repeat sequence may be a synthetic polynucleotide sequence and its inverted repeat sequence or an inverted repeat of all or part of the said gene silencing vector or an inverted repeat of the 5'-untranslated region of the gene silencing vector.

The inverted repeat may be separated from the polynucleotide region by a sequence of nucleotides.

The invention also provides a method of controlling the expression of a DNA sequence in a target organism, comprising inserting into the genome of said organism an enhanced gene silencing vector as defined above.

In a preferred embodiment a vector for enhanced gene silencing comprising in sequence a promoter region, a 5'-untranslated region, a transcribable DNA sequence and a 3'-untranslated region containing a polyadenylation signal, characterised in that the said construct includes an inverted repeat of a region of said vector.

It is preferred that the inverted repeat is a fragment of the 5'-untranslated region of the said vector. The vector may have two tandem copies of the inverted repeat.

In simple terms, we have found that the inhibitory effect of a gene-silencing vector can be enhanced by creating in the vector an inverted repeat of a part of the sequence of the vector. Alternatively the inverted repeat may be of a synthetic sequence which may be created independently of the vector itself and then inserted into the vector sequence. While the mechanism by which the enhancement is achieved is not fully understood we understand that the minimum required for such a vector is a region or regions which identify the gene targeted for silencing and an inverted repeat of a part of that region or, as explained above an inserted sequence and its inverted repeat. The region of the vector which identifies the gene targeted for silencing may be any part of that endogenous gene which characterises it, for example, its promoter, its 5'-untranslated region, its coding sequence or its 3'untranslated region. We have also found that the vector used in this invention will silence the expression of the target gene and also any members of the gene family to which the targeted gene belongs.

Although the mechanism by which the invention operates is not fully understood, we believe that creation of an inverted repeat promotes the formation of a duplex DNA between the selected sequence and its inverted.

The inverted repeat may be positioned anywhere within the vector such as within the promoter region, the 5' untranslated region, the coding sequence or the 3' untranslated region. If the inverted repeat is based on a contiguous sequence within the promoter region, then it is preferred that the inverted repeat in located within the promoter region. If the inverted repeat is based on a contiguous sequence within the 5' untranslated region, then it is preferred that the inverted repeat is located within the 5' untranslated region. If the inverted repeat is based on a contiguous sequence within the coding region, then it is preferred that the inverted repeat is located within the coding region. If the inverted repeat is based on a contiguous sequence within the 3' untranslated region, then it is preferred that the inverted repeat is located within the 3' untranslated region.

The selected polynucleotide sequence and its inverted repeat may or may not be separated by a polynucleotide sequence which remains unpaired when the 5' untranslated region and the inverted repeat have formed a DNA duplex. It is preferred however, that the chosen contiguous sequence and its inverted repeat are separated by a polynucleotide sequence which remains unpaired when the 5' untranslated region and the inverted repeat have formed a DNA duplex.

It is further preferred that the inverted repeat is based on the 5' untranslated sequence. It is also preferred that the inverted repeat is positioned upstream of the coding sequence. It is further preferred that the inverted repeat is positioned between the 5' untranslated region and the coding sequence. It is further preferred that the 5' untranslated region and the inverted repeat are separated by a polynucleotide sequence which remains unpaired when the 5' untranslated region and the inverted repeat have formed a DNA duplex.

Suppression can also be achieved by creating a vector containing an inverted repeat sequence which is capable of forming a duplex DNA within the promoter region of the target gene. This obviates the need to include any specific coding sequence information about the gene to be suppressed since the vector would allow suppression of the promoter within the organism and hence the expression of the target gene. Alternatively vectors may be created which are lacking a promoter sequence but which contain an inverted repeat of a sequence within the 5' untranslated region, the coding region or the 3' untranslated region.

The 5' or 3' untranslated regions of a gene suppression vector can also be replaced with a synthetic 5' or 3' untranslated regions which comprises a polynucleotide part and inverted repeat separated by a polynucleotide sequence which remains unpaired when the said polynucleotide part and the inverted repeat form a DNA duplex. It is preferred to construct a synthetic 5' untranslated region. It is further preferred to construct the synthetic 5' untranslated region comprising sequentially, a 33 base polynucleotide part and a 33 base polynucleotide inverted repeat separated by a 12 base polynucleotide.

Where it is desired to use an inverted repeat sequence within the 5' untranslated region, the coding sequence or the 3' untranslated region, gene silencing vectors constructed with inverted repeats within any one of these regions may additionally enable the silencing of genes that are homologous to the coding sequence present in the silencing vector. Therefore when it is desired to silence genes homologues within an organism the construction of a silencing vector containing an inverted repeat within the 5' untranslated region, the coding sequence or the 3' untranslated region may allow the silencing of all the genes exhibiting sequence homology to the coding sequence within the construct. Homology/homologous usually denotes those sequences which are of some common ancestral structure and exhibit a high degree of sequence similarity of the active regions. Examples of homologous genes include the ACC-oxidase enzyme gene family which includes ACO1 and AC02.

Any of the sequences of the present invention may be produced and manipulated using standard molecular biology techniques. The sequences may be obtained from a desired organism source such as plant sources and modified as required or synthesised *ab initio* using standard oligosynthetic techniques.

Without wishing to be bound by any particular theory of how it may work, the following is a discussion of our invention. 96% of tomato plants transformed with an ACC-oxidase sense gene containing two additional, upstream inverted copies of its 5' untranslated region, exhibited substantially reduced ACC-oxidase activity compared to wild type plants. Only 15% of plants transformed with a similar construct, without the inverted repeat, had reduced ACC-oxidase activity. Both populations had similar average numbers of transgenes per plant. Treatment of tomato leaves with cycloheximide caused a strong, reproducible increase in the abundance of ACC-oxidase transcripts and was used in the study of suppression by ACC-oxidase sense transgenes in preference to wound induction used in previous studies. The relative abundance of unprocessed and processed ACC-oxidase transcripts in suppressed and non-suppressed plants was assayed by ribonuclease protection assays, providing an indirect measure of transcription and mRNA accumulation which did not rely upon assaying isolated nuclei. This analysis indicated that the suppression of ACO1 gene expression was mainly post-transcriptional. Using the same type of RPA assay similar results were obtained from plants containing suppressing polygalactronase-sense or ACO-antisense transgenes.

There are now numerous examples of the inactivation of homologous sequences in plants. The term "homology dependent gene silencing" (HDGS) best describes all of these although it should be noted that in most examples the "silencing" is not complete and a low level of gene expression remains. Throughout this specification we will use the classification most-recently outlined by Matzke and Matzke, Plant Physiol. 107: 679-685 (1995) in which different examples of HDGS were divided into three main groups; cis-inactivation, trans-inactivation, and sense-suppression. Down regulation by antisense genes bears many similarities to the last of these and has been suggested to operated by the same mechanism (Grierson et al, Trends Biotechnol. 9: 122-123 (1991)). Both sense and antisense transgenes have been widely used to reduce the expression of homologous endogenous genes in plants. Although the underlying mechanisms of HDGS remain obscure, this technology has found numerous applications not only in fundamental research but also in commercial biotechnology ventures and new food products are already on the market.

At present , obtaining a large number of strongly suppressed, transgenic lines is more a matter of luck than judgement. A positive correlation between the presence of repeated transgene sequences and the incidence of HDGS has been noted. However single locus-transgene insertions associated with HDGS have also been reported.

There is an emerging consensus that different examples of HDGS can be classified on the basis of whether or not the transcription of the target gene is affected. Examples of transcriptional suppression have been described. Where the homology between interacting genes resides within transcribed sequences, HDGS has been shown to be a post-transcriptional effect. Despite this apparently precise demarcation, several similarities exist between some examples in the two different categories. These include variegated patterns of silencing, increased methylation of genes participating in silencing and the frequent observation that silencing loci contain repeated sequences.

Although transcriptional silencing must occur in the nucleus, post-transcriptional silencing might occur in either or both the nucleus or cytoplasm. There is evidence that the abundance of processed, nuclear RNA of silenced genes was unaffected and suggested an effect upon transport into or degradation within the cytoplasm. More compelling evidence that post-transcriptional HDGS occurs outside the nucleus is the relationship between gene silencing involving nuclear transgenes and resistance to cytoplasmically replicating RNA viruses. Transgenic plants containing transgenes that suppress the activity of other transgenes (e.g. GUS) or endogenous genes (e.g. PG) are also resistant to RNA viruses which have been engineered to include sequences from those genes. Nevertheless, nuclear features such as transgene methylation and complexity of transgene loci were found positively to correlate with virus resistance. In almost all instances of HDGS, the source of the silencing is nuclear (even if the manifestation is cytoplasmic). However, silencing of a nuclear gene by a cytoplasmic element has been demonstrated by the suppression of phytoene desaturase in plants infected by a recombinant virus containing sequences from that gene.

Although, there are now numerous examples of post-transcriptional suppression of plant genes by HDGS, as yet, there is no information as to whether the increased turnover of pre-mRNA is related to or distinct from other cellular, RNA turnover processes.
Degradation of RNA in plants is poorly understood but there is evidence that translation is involved. For example, the very short half lives (around 10 minutes) of small auxin up RNAs (SAURS) can be markedly prolonged by treatment with cycloheximide.

This invention gives a striking increase in the frequency of HDGS following the inclusion of a short repeated region within a transgene. Expression of the target gene encoding the terminal ethylene biosynthetic enzyme ACC-oxidase, in tomato was suppressed by such constructs mainly post-transcriptionally. This was shown to be true for other examples of sense and antisense suppression in tomato. Cycloheximide was found to be a potent and reliable inducer of ACO gene expression but did not ameliorate the silencing.

The invention will now be described, by way of illustration, in the following Examples and with reference to the accompanying Figures of which:
**Figure 1.** (A) ACO1 gene silencing vector. (B) ACO1 gene silencing vector containing tandem inverted repeats of the 5' untranslated region.
**Figure 2.** Illustrates the relative ACC-oxidase activity in both types of transgenic plant relative to wild type values where C = transgenic plants containing construct C (Figure 1A) and V = transgenic plants containing construct V (Figure 1B).

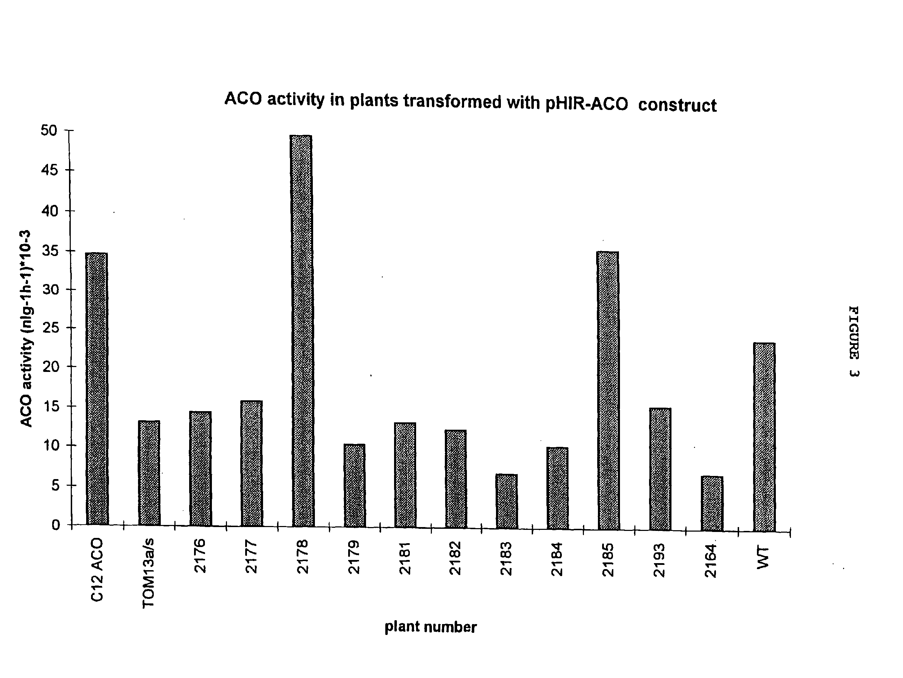

**Figure 3.** Tomato plant ACC Oxidase activity of transgenic transformants containing pHIR-ACO (as illustrated in SEQ ID No 10). The graph also includes C12ACO (overexpression control) an untrasformed wild type and TOM13 strong antisense gene silenced control.

### Example 1.0

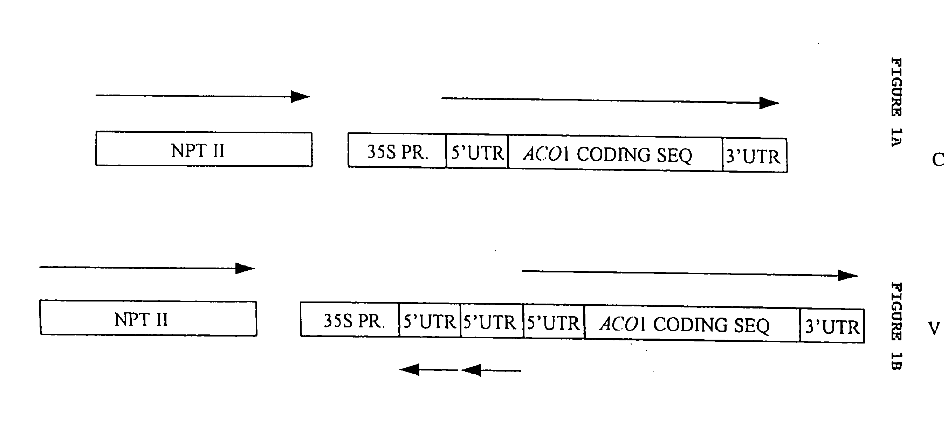

Construct V (Figure 1) was made in the following manner: 79 base pairs of the 5' untranslated region of the tomato ACO1 cDNA was amplified by PCR and two copies were ligated in tandem in the reverse orientation immediately upstream of the ACO cDNA which contains its own polyadenylation signal in its 3' untranslated region (construct C). Both were ligated downstream of the CaMV 35S promoter and then transferred to the binary vector, Bin19. Figure 1 shows the basic details of constructs "C" and "V". These were used to transform tomato plants (Ailsa Craig) by *Agrobacterium* mediated DNA transfer. 13 and 28 individual kanamycin resistant calli were obtained with constructs "C" and "V" respectively and these were regenerated into plants.

The nucleotide sequence of the promoter and 5' untranslated region of the ACO1 gene is given as SEQ ID NO 1 hereinafter. The 79bp referred to above begins at base number 1874 and stops at the base immediately preceding the translation start codon (ATG) at number 1952.

### Example 1.1

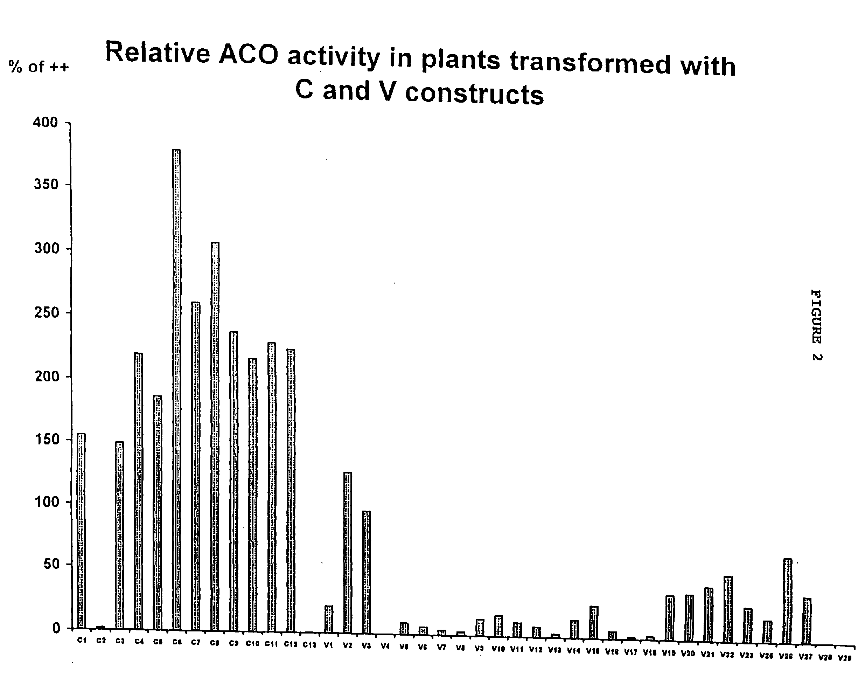

To screen the population for any effects on ACO gene expression, relative ACO activity was measured from untransformed and transformed plants. The production of ethylene from leaf discs supplemented with the ethylene precursor, 1-aminocyclopropane-1-carboxylic acid, was measured at least three times from each plant. The cutting of the discs by a cork borer wounds the leaves and stimulates the expression of the ACO1 gene. ACC-oxidase activity in both types of transgenic plant relative to wild type values are shown in Figure 2. There was a dramatic difference in ACO activity between the two populations, with plants containing the inverted repeat (V line) showing very strong suppression. The majority (11 out of 13) of plants of the C line did not show suppression of ACO activity but overexpression, compared to wild-type plants, as would be expected since this construct contained a translatable ACO1 coding sequence.

To test for the presence of the transgenic ACO sequence, DNA from the plants was analysed by PCR using two oligos homologous to and complementary with the beginning and end respectively of the ACO1 coding sequence. This combination co-amplifies 1500 bp of the endogenous ACO1 gene (which acts as an internal positive control) and the ACO1 sense transgene as a 1000 bp fragment (since it was derived from a cDNA and so has no introns). The amplified region does not include the repeated region of the V-type transgene. The two fragments were separated by gel electrophoresis and detected by staining with ethidium bromide. This showed the presence of the transgene in all plants of the C line and all plants of the V line except one (V2) which also had no reduced ACC-oxidase activity (Figure 2).

### Example 1.2

It was considered possible that the repeated region in the transgene might have affected the number of transgenes which integrated into the genome and that this was the actual source of high frequency silencing. The PCR assay described above can be used to estimate the transgene copy number if the following assumptions are made:
- 1): that in any transgenic plants there was no variation in the number of endogenous ACO1 genes per genome;
- 2): that the amplification efficiency ratio (endogenous ACO1 DNA: transgenic ACO1 DNA) is constant;
- 3): the reaction is sampled at low DNA concentration to minimise product re-annealing. Since we were only concerned with estimating the number of transgenes in the two lines relative to each other and not absolute quantification of transgene copy number, we did not employ synthetic combinations of "transgene" and "endogenous gene" DNA as standards.

After 20 cycles of amplification, gel-electrophoresis, Southern blotting, and hybridisation with a radioactively labelled ACO1 cDNA, the signal from endogenous and transgenic ACO1 DNA was visualised and quantified by phoshorimaging. The average transgene: endogenous gene ratio for the C line was 0.96 and for the V line 1.08 indicating that the repeat region in the V construct does not cause more T-DNAs to integrate during transformation.

### Example 1.3

ACO1 mRNA increased in abundance following wounding and/or treatment of leaves with cycloheximide but accumulation was approximately five times greater after treatment with cycloheximide than after mechanical wounding which we have previously used as a stimulus. Wounding of cycloheximide treated leaves failed to elicit a further increase in ACO1 mRNA amount. We found cycloheximide to be a more reproducible inducer of ACO1 mRNA accumulation than mechanical wounding and so have used it in preference to the latter in this study. No further increase in the abundance of ACO1 mRNA was observed when the concentration of cycloheximide was increased from 50 to 250 ug/ml (date not shown).

### Example 1.4

The 5' end of ACO1 mRNA extracted from plants is heterogeneous but consists of two major species which differ by 2 bases. The 5' untranslated region (both the sense and duplicated antisense sequences) in both of the constructs (C and V) was made approximately 10 base pairs shorter than those of the endogenous gene. This allowed the discrimination of endogenous gene and transgene-derived transcripts by ribonuclease protection assays using a probe transcribed from a genomic ACO1 sequence which extended from the start of the 3' end of the 5' untranslated region to a Accl site, in the promoter of ACO1, 222 bases upstream. In RNA from wild type leaves, there were several bands which may arise from distinct RNA species or from breaking of RNA duplexes during digestion. Some of the bands seem more susceptible to the effects of antisense suppression than others (although the general trend is still suppression).

In leaves from lines V4, V11 and V28 (all <10% ACO activity), there was extensive co-suppression of the endogenous transcripts (relative to wild-type) and the transgene transcripts (relative to those from a control transgene (line C1). V4, V11 and V28 all exhibited greater suppression than the homozygous ACO-antisense line (Hamilton et.al. Nature 346, 284-287(1990)).

The use of the protein synthesis inhibitor cycloheximide as a stimulant of ACO1 RNA accumulation did not obviously alleviate the suppression of this RNA by the sense transgenes in lines V4, V 11 or V28.

Although the endogenous genes transcript is unquestionably suppressed, it is possible that the inverted repeat within the 5' end of the V transgene transcript excludes the probe and causes the signal from the transgenic RNA to be underrepresented. This seems unlikely for the following reason. When a probe that was not excluded by the inverted repeat was used to analyse RNA from the V line, the mRNA signal (which, using this probe, is actually the sum of the endogenous and the transgenic RNAs) was still much less than in the wild type. The data shows that in the absence of silencing, the abundance of the endogenous and transgenic RNAs are comparable.

### Example 1.5

We chose to measure the abundance of unprocessed transcripts in total RNA extracts as a indirect measurement of transcription whilst simultaneously measuring the amount of processed mRNA. This was achieved using RNA probes transcribed from genomic sequences spanning introns in ribonuclease protection assays. Since the RNA analysed was from leaves frozen in liquid nitrogen and then extracted in strongly protein-denaturing conditions (phenol and detergent) there should have been little opportunity for any resetting of transcription during the process There was a greater abundance of mRNA following treatment with cycloheximide although the total amount of mRNA in the ACO-AS plants was reduced. In the ACO-sense line, V11, there was little or no increase in the mRNA signal. It is likely that this mRNA signal is mainly from the transgene which is transcribed by the 35S promoter which is not cycloheximide inducible. In contrast, the abundance of the primary transcript in all RNA samples increased following cycloheximide treatment. This RNA species originates only from the endogenous ACO1 gene since the transgene has no introns. In all cases the suppressing transgene had little or no effect upon the abundance of the primary transcript.

### Example 1.6

Cycloheximide strongly stimulated the accumulation of both the ACO1 primary transcript and mature mRNA. Quantification of the signal from primary transcripts and mature ACO1 RNA in wild type leaves before and after treatment with cycloheximide showed that there was a 6 fold increase in the abundance of unprocessed ACO1 RNA but a 13 fold increase in the amount of processed ACO1 RNA. The abundance of transgenic ACO1 RNA (transcribed from the 35S promoter) in the C line also rose upon treatment with cycloheximide.

### Example 1.7

Two tandemly linked copies of the 5'UTR (each unit = 79bp; 74.7% (A+T)) were litigated in the inverted orientation between the CaMV 35S promoter and an almost full length ACO1 cDNA (Figure 1). Either unit of this direct repeat has the capacity to form a large cruciform structure with the 5'untranslated region immediately downstream. After *Agrobacterium-*mediated transformation with this construct, 26 out of 28 plants recovered from tissue culture exhibited suppressed ACO activity. A much lower frequency (2/15) of suppression was observed with a control construct which lacked the duplicated 5'UTR but was otherwise the same.

More transgenic plants were obtained with the V construct than with the control construct (as well as exhibiting the high HDGS frequency). It is likely that this is a direct result of reduced ethylene synthesis as a result of ACO gene suppression. Previous results have shown that greatly improved callus regeneration could be achieved after transformation with constructs which contained an ACO-antisense gene.

Of the two plants transformed with the repeat construct that showed no suppression, one, V2, may have had a truncated T-DNA or be an untransformed escape since the transgenic ACO1 sequence could not be amplified. Since the repeat contained DNA sequences already in the gene, it seems unlikely that it is this sequence per se which elicits the effect upon gene silencing. It is much more likely that it is the structure of the repeat DNA (or the transcribed RNA) which is the source of the high frequency of silencing observed. The repeat within the V construct was similar to that with the control construct

Most instances of HDGS are associated with complex transgenic loci that contain repeats or whole or part T-DNAs rather than simple single insertions but it is not known whether this is a primary determinant of suppression or an indirect effect. There are examples where apparently single transgenes are associated with gene silencing but these are in the minority and in at least some of these examples the T-DNAs contain internal repeats. The data presented here suggest that deliberate introduction of small repeats in a transgene can increase the number of transgenic lines in which homologous genes have been suppressed to almost 100%. Sense suppression could be obtained with the control construct but at a much lower frequency. The deliberate introduction of repetitive DNA into a transgene may substitute for a requirement for the insertion of repeated T-DNA units to produce silencing. Although the PCR assay used here is not absolutely quantitative, it does suggest that the average transgene dosage is about 2 implying that some of the lines exhibiting suppression have single insertions. In several of our lines, the suppression obtained is profound (Figure 2) which makes this strategy even more attractive to those interested in specifically switching off gene expression. There is one previous report of the deliberate combination of repetitive DNA with a reporter gene effecting increased HDGS: Lohuis et al., Plant Journal, 8, 919-932 (1995) inserted a copy of a randomly isolated repetitive genomic sequence (RPS) upstream of GUS reporter gene and found that this element increased the frequency of variegation of transgene expression. This is an example of cis-inactivation, probably acts at the transcriptional level, and the authors considered it to be distinct from co-suppression/sense-suppression phenomena. Interestingly, the RPS element did not increase the frequency of complete silencing of the transgene. In our example, although the level of suppression is severe in many lines, it is not possible to say whether the degree of suppression is equal in all cells expressing the target gene or if the repeat has simply greatly increased the proportion of cells experiencing suppression.

### Example 1.8

### Constructs and transformation

The tomato ACO1 cDNA, pTOM13 was released from its original cloning vector, pAT153, (Promega), creating pG31. pG31 was digested with EcoRI and the vector religated to create pTRD. This removed the 5' end of the cDNA which contains approximately 90 base pairs of the 3'untranslated region in the antisense orientation at its 5' end which may have been introduced artefactually during the original cloning of the pTOM13 cDNA . The remaining ACO1 sequence was cut out from pTRD with EcoRI and Hindlll and ligated into pT₇.T_{3α}18 (BRL) digested by EcoRI and the ends filled in with Klenow enzyme. The 5' untranslated region of the ACO1 transcript (minus approximately 10 bases at the 5' end) was amplified with Taq polymerase from oligo dT-primed cDNA of wounded tomato leaves with the primers 5' CATTCATCTCTTCAATCTTTTG 3' (SEQ ID No.2) and 5' CTTAATTTCTTGGTAAAGTGTTTTCC 3' (SEQ ID NO.3). This DNA was rendered flush ended with T4 DNA polymerase and ligated with the filled in pTRF to create pMl1. This reconstituted the EcoRI site at the 5' end and yielded a translatable ACO1 cDNA slightly shorter than the wild type ACO1 mRNA. Sequencing confirmed that the amplified ACOI sequence was not mutated. pMI1 was digested with HindIII and partially with EcoRI and the fragment containing the ACO1 cDNA sequence was filled in with Klenow enzyme, and ligated with Smal digested pDH51 to create pDHC1. This was digested with Xbal and HindIII, the filled in and the fragment containing the vector, 35S promoter and ACO1 cDNA religated to create pMI5. pMI7 contains two copies of the 5'UTR of ACO1 tandemly linked and inserted in the antisense orientation upstream of the 5'UTR of ACO1 in pMI5. This was made by amplifying the 5'UTR from tomato leaf cDNA (see above) with oligos 5' CATTCATCTCTTCAATCTTTTG 3' (SEQ ID No.2) and
5'CTTAATTTCTTGGTAAAGTGTTTTCC 3'(SEQ ID NO.3)., polishing the DNA with T4 DNA pol and ligating it into a filled in Acc651 site in pMI5 upstream of the 5'UTR of the ACO1 sequence Acc651 (an isoshizomer of Kpn1 but which gives a 5' overhang). The construction was confirmed by sequencing.

pDHC1 and pMI7 were digested with BamHI, BgII and PvuII and the BamHI-PvuII fragments containing the CamV35S-AC01cDNA sequences were cloned into Bin19 which had been cut by HindIII, filled in and then cut by BamHI. The resulting recombinants were called pBC1 and pBM17 respectively. These plasmids were transformed into *A. tumefaciens* LBA4404: and this used to transform tomato cotyledons *(Lycopersicon esculentum* var Ailsa Craig). Plants were regenerated from callus grown on 50µg.ml⁻¹ kanamycin.

### Example 1.9

### ACC-oxidase assays

ACC-oxidase activity was measured as the ability of plant tissue to convert exogenous 1-aminocyclopropane-1-carboxylic acid (ACC) to ethylene. Discs were cut from leaf lamina with a sharp cork borer and placed in contact with 0.5ml of 10mM NaH₂PO₄/Na₂HPO₄ (pH7), and 10 mM ACC (Sigma) in 5 ml glass bottles which were then sealed with "Subaseal" vaccine caps (Fisons). After 1 hour at room temperature, the ethylene in the head space was measured by gas chromatography as described by Smith et al., 1986. Ethylene was also measured from bottles containing the solution but without leaf tissue. These values were subtracted from the values obtained from the bottles containing leaf discs.

### Example 1.10

### PCR analysis of transgenic plants

DNA was extracted from singles leaves of wild type plants, plants homozygous for a ACO-antisense gene, and those transformed with the constructs of pBCl and pBM17. Leaves were frozen in liquid nitrogen, briefly ground in eppendorf tubes with a disposable pipette tip, ground further after the addition of 200µl DNA extraction buffer (1% laurylsarcosine, 0.8% CTAB, 0.8M NaCl, 0.02M EDTA, 0.2M Tris/HCI (pH8)), heated to 65°C for 15 minutes, extracted once with phenol/chloroform and the DNA precipitated from the aqueous phase by the addition of 0.6 volumes of isopropanol. The DNA was recovered by centrifugation, the pellets washed in 70% ethanol, dried and redissolved in 200ul, of TE buffer. 1ul of this was used as template for simultaneous PCR amplification of the endogenous ACO1 gene and the transgene using the primers ACO1.1 (ATGGAGAACTTCCCAATTATTAACTTGGAAAAG SEQ ID NO 4) and the ACO1.2 (CTAAGCACTTGCAATTGGATCACTTTCCAT SEQ ID NO 5) for 21 cycles of 30 seconds at 95°C, 30 seconds at 65°C and 1 minute at 72°C. Amplified DNA was separated by electrophoresis in a 0.8% agarose/1xTBE gel and blotted onto HybondN+ in 0.4M NaOH for 6 hours. To detect the amplified ACO sequences, the DNA on the filter was hybridised with random prime labelled ACO1 cDNA. The filter was washed in 0.2xSSPE/1%SDS at 65°C followed by phosphorimaging of the radioactive signal.

### Example 1.11

### Treatment of leaves with cycloheximide and mechanical wounding

Compound leaves were excised with a sharp scalpel blade and immediately placed under water solution of 50µl.ml⁻¹ cycloheximide (Sigma). Another 3 cm of the stalk was cut from the branch under the solution and the assembly was then left in a laminar airflow for six hours to allow the cycloheximide to enter the leaves.

To wound leaf tissue, individual leaflets were placed on a hard surface and diced with a sharp scalpel blade approximately 10 times transversely and 5 times longitudinally.

### Example 1.12

### Northern analysis of ACO mRNA in leaves treated with cycloheximide

RNA was extracted from cycloheximide treated leaves as follows. Tissue was frozen in liquid nitrogen and pulverised either in a coffee grinder (for fruit pericarp, see below) or in a mortar (for leaves). 5ml.gfwt⁻¹ of RNA extraction buffer (Kirby's) was added and the frozen slurry ground further in disposable polypropylene centrifuge tubes with a glass rod. Once thawed, the mixture was extracted twice with phenol/chloroform and the nucleic acids precipitated by the addition of 2.5 volumes of ethanol, 1/10 volume 3M sodium acetate (pH5) and refrigeration at 20°C for 1 hour. After centrifugation at 3000xg for 10 minutes (40 minutes for a fruit extraction), the pellets were redissolved quickly in water (approximately 1ml per gram of tissue) and, an equal vol. of 2x DNA extraction buffer (1.4M NaCl 2% CTAB, 100mM Tris/HCl (pH8)). Two volumes of precipitation buffer (1%CTAB, 50mM Tris/HCl (pH8)) were added to precipitate the nucleic acids (30 minutes at room temparature suffices) and the precipitate was collected by centrifugation (3000xg/15 minutes). This step was repeated except the pellets were dissolved in 1xDNA extraction buffer. After collection of the second precipitation, the pellets were redissolved in 0.5ml 1M NaCl and immediately reprecipitated with 2.5 volumes of ethanol (-20°C/30 minutes). After centrifugation (10000xg/10 minutes), the pellets were redissolved in 400µl water and extracted twice with phenol/chloroform. The nucleic acids were precipitated and collected as above redissolved in 400µl water. 46ul of 10 x One-Phor-All-Buffer (Pharmacia) was added with 50 units of RNAase-free DNAase (Promega) and the solutions incubated at 37°C for 30 minutes. They were extracted twice with phenol/chloroform, the RNA precipitated and collected as above and finally redissolved in 100-500ul of water. We have found that this relatively extensive purification is necessary if rare transcripts are to be detected by RPA. Also, the RNA redissolves readily which greatly reduces handling time when manipulating this RNA mixed with radioactive probe RNA.

50µg of leaf RNA was mixed with an equal volume of denaturation/loading solution (50% formamide; 25mM sodium phosphate (pH6.5); 10mM EDTA; 6.2% formaldehyde; 200µg.ml⁻¹ ethidium bromide) and separated by electrophoresis on a 25mM sodium phosphate (pH6.5) /3.7% formaldehyde /1.5% agarose gel in 10mM sodium phosphate (pH6.5)/3.7% formaldehyde with continuous buffer re-circulation. The separated RNA was blotted onto Genescreen (Dupont) hybridisation membrane in 10mM sodium phosphate (pH6.5). The autocrosslink setting on a Stratalinker (Stratagene) was used to covalently link the RNA to the filter. The filter was prehybridised and then hybridised with a 32P-random prime labelled ACO1 cDNA probe. The filter was washed in 0.2xSSPE/1%SDS at 65°C and then exposed to Kodak X-omat film between two intensifying screens at -70 for 24 hours. Subsequently the radioactivity in each band was measured by phophorimaging.

### Example 1.13

### Ribonuclease protection analysis

RNA was extracted from cycloheximide treated leaves and fruit described above.

RNA probes were transcribed with T7 RNA polymerase at 20°C with α-³²P UTP (400Ci. mmol⁻¹) as the sole source of UTP. After 1 hour incubation, RNAase-free DNAase was used to remove the template and the probe was further purified on 6%polyacrylamide/8M urea/1xTBE gels. The band containing the full length probe was visualised by autoradiography. The gel slice containing this RNA was excised and placed in 1ml probe elution buffer (0.5M ammonium acetate; 1mMEDTA; 0.2% SDS) for between 6 and 14 hours at 37°C. Typically, between 20m and 100µl of this would be co-precipitated with between 20 or 100µg of the RNA to be tested plus two yeast RNA controls. The precipitated RNAs were redissolved in 30µl hybridisation solution (80% formamide; 40mM PIPES/NaOH; 0.4M sodium acetate; 1mM EDTA pH should be 6.4) heated to 65°C for 10 minutes and hybridised at 42°C for between 2 to 14 hours. The longer hybridisation times were purely for convenience since we easily detected even rare transcripts after only 2 hours of hybridisation. 300µl of RNAase digestion buffer (5mM EDTA; 200mM sodium acetate; 10mM Tris/HC1. Final pH of solution should be 7.5) containing either RNAaseONE (Promega) or RNAase T1 (Ambion) was added to each tube except one containing yeast RNA which received RNAase digestion buffer without any ribonuclease. Incubation of the digesting RNA was at either 25°C (RNAaseONE) or 37°C RNAaseT1) for 2-4 hours. RNAaseONE was inactivated by the addition of SDS to 0.5% and the protected, double stranded RNAs were precipitated with ethanol and sodium acetate. RNAaseT1 was inactivated and the double stranded RNAs were precipitated by the addition of the inactivation/precipitation solution provided with the RNAase protection kit from Ambion. The protected RNAs were redissolved in 5-10ul of denaturation/loading solution (80% formamide; 10mM EDTA; 0.1% bromophenol blue; 0.1% xylene cyanol; 0.1% SDS), heated to 95°C for 5 minutes and then separated by electrophoresis on a on 6-8% polyacrylamide/8M urea/1xTBE gels (the concentration of polyacrylamide depending on upon the sizes of the fragments to be separated). After electrophoresis, the gels were dried and exposed to Kodak x-omat film between two intensifying screens at -70 for the time indicated. The radioactivity was measured by phosphorimaging.

### EXAMPLE 2.0

### Construction of synthetic heterologous DNA inverted repeat.

A synthetic heterologous DNA invert repeat (SEQ ID No 11) was constructed by annealing two sets of synthetic oligos (HIR1 SEQ ID No 12 and HIR2 SEQ ID No 13 and HIR 3 SEQ ID No 14 and HIR 4 SEQ ID No 15) and ligating each set into pSK-(bluescript, Statagene) independently, to create pHIRA and pHIRB respectively. The invert repeat structure was created by digesting both pHIRA/B vectors with Xhol and Ncol and ligating the 42bp fragment from pHIRB into the pHIRA. The invert repeat structure was isolated from the pSK- vector using KpnI and cloned into the KpnI site immediately downstream of the CaMV35S promoter in the plant expression cassette pSIN to create pHIR-SIN.

The tomato ACO1 cDNA (pTOM13) coding sequence was amplified from its original cloning vector pAT153 (promega) using two oligonucleotide primers, 5' CTTTACCAAGAAGTGCACATGGAGAACTTCCC 3'SEQ ID No 6, and 5'GAATTGGGCCCTAAGCACTTGCAATTGG 3' SEQ ID No 7 which prime either side of the TOM13 coding sequence introducing ApaLI and Apal sites respectively. The PCR product was digested with ApaLI and Apal and the ends blunted in using Pfu polymerase (Stratagene). The blunt PCR fragment was ligated into the SmaI site downstream of the invert repeat structure of pHIR-SIN to create pSIN-HIR-ACO.

The plant expression cassette from pHIR-ACO was isolated using Agel and ligated into the binary vector pVB6 Agel site to create pHIR-ACO SEQ ID No 10. The insert was orientated using restriction analysis to ensure that all the ORF that will be active in the plant were unidirectional. pHIR-ACO was transformed into *A.tumafaciens* LBA4404: and this used to transform tomato cotyledons *(Lycopersicum esculentum* var Ailsa Craig). Plants were regenerated from callus.

### Example 2.1

### Identification of Transgenic Plants

DNA was extracted from single leaves and extracted as described previously. Plants containing the HIR-ACO T-DNA insert were identified by PCR using an internal TOM13 sense primer (5' GCTGGACTCAAGTTTCAAGCCAAAG 3'SEQ ID No 8) and a NOS 3'UTR (untranslated region) specific antisense primer
(5'CCATCTCATAAATAACGTCATGC3' SEQ ID No 9)

### Example 2.2

### ACC-oxidase assays

ACC-oxidase activity was measured as the ability of plant tissue to convert exogenous 1-aminocyclopropane-1-1carboxylic acid (ACC) to ethylene. Small leaves were removed from shoots and wounded with a scalpel before being placed into a 2ml sealable vial, and left for 30minutes. The vials were then sealed and left for an hour at room temperature , after which the ethylene in the head space was measured by gas chromatography as described my Smith et al., 1986. Ethylene was also measured from wildtype, over-expressing (C12) and antisense down-regulated plant material.

## Claims

1. A polynucleotide for silencing expression of a selected target DNA, comprising a first sequence of nucleotides and a second sequence of nucleotides in which the nucleotides of said second sequence are complementary to and in reverse order to those of the first such that first and second sequences are capable of forming a duplex polynucleotide, and said polynucleotide includes a sequence of nucleotides identifying the target DNA.

2. A polynucleotide as claimed in claim 1, in which said first and second sequences are synthetic polynucleotides.

3. A polynucleotide as claimed in claim 1, in which first and second sequences are the 5'-untranslated region of the target DNA.

4. A polynucleotide as claimed in any of claims 1 to 3, in which the first and second sequences are separated by a sequence of nucleotides.

5. A vector for silencing expression of a target DNA within an organism comprising in sequence a promoter region, a 5'-untranslated region, a transcribable DNA sequence and a 3'-untranslated region containing a polyadenylation signal, in which the transcribable DNA sequence is a polynucleotide as claimed in any one of claims 1 to 4.

6. A vector as claimed in claim 5, in which the said transcribable region comprises in sequence from 5' to 3', a first DNA complementary to the 5'-untranslated region of the target DNA, a copy of said first DNA, and, a third DNA comprising the complement of said first DNA the nucleotides of which are in reverse order to the first sequence such that said transcribable region is capable of forming a duplex polynucleotide..

7. The transcription product of a vector as claimed in claim 5 or claim 6.

8. A method of silencing the expression of a DNA sequence in a target non-animal organism, comprising inserting into the genome of said organism a polynucleotide as claimed in any of claims 1 to 4.

9. A method of silencing the expression of a DNA sequence in a cell of a target organism, comprising inserting into said cell a polynucleotide claimed in any of claims 1 to 4.

10. A cell of a target organism comprising a polynucleotide as claimed in any of claims 1 to 4.


SEQUENCE LISTING

(1) GENERAL INFORMATION:

(i) APPLICANT:
(A) NAME: SYNGENTA LIMITED
(B) STREET: EUROPEAN REGIONAL CENTRE, PRIESTLY ROAD
(C) CITY: GUILDFORD
(D) STATE: SURREY
(E) COUNTRY: UNITED KINGDOM
(F) POSTAL CODE (ZIP): GU2 7YH
(G) TELEPHONE: +44 1344 414822
(H) TELEFAX: +44 1344 481112
(I) TELEX:

(ii) TITLE OF INVENTION: GENE SILENCING

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER:

(2) INFORMATION FOR SEQ ID NO: 1:

(i) SEQUENCE CHARACTERISTICS:
(A) LENGTH: 3681 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA"

(vii) IMMEDIATE SOURCE:
(B) CLONE: SEQ ID NO 1

(xi) SEQUENCE DESCRIPTION: SEQ ID NO: 1:

AAATTTGATA GATTCAGTTT TTATGTTTTT AGTGCTGATT ACAACATTGA AATTCTAAAT 60

TTAGAATTTA ATATTTATTA AATGTTAGTG CATTTATACA AATAACATAT TACATCTCAA 120

ATAATATTGA GTTTGTTAGA TTTTATTTGC CCTGATTTCT TATCATAAAT AGGTTTTCCT 180

TTTAGGAAAA GGTTTTGAAT TGACTATTCT TTTTTTGGTA GGAAAAAGTT TAGGACTCTA 240

TAAATAGAGG CATGTTCCTT CTAACTTAAT TAGCATTCAC AATGTAGTTT TAAGGGCTTT 300

GAGAGTTTTG GTTAGAGGGA GAATTTGTGA ACCTCTCATG TATTCCGAGT GAATTGGTTG 360

AGGTTGTTTC CCTCTGTATT TTGTACTCTC ATGTTTATAG TGGATTGCTC ATTTCCTTTG 420

TGGACGTAGG TCGATTGACC GAACCACGTT AAATTTTTGT GTCTTTTGGT ATATTTCCTG 480

TTCTTCTTAC TCGTGGTCTT TCGAGGTTTG CTTTGCTAGC TTCCGCGTTT ACACCTGCTT 540
ATTTTCGGTC CTAACAAGTG GTATCAGAGC CAGATTCAAT AATGGAGTCA GGTGTAGTGG 600
TTCGATAATC GATGATTGAA CCAAGTTAGA AAGAGGTGTT CATCTTGACG GGTGTAGTTC 660
TAGCCGCAAC CTTTTTGACA GTAATGAAGA TTTTGATGGA GAAATTGTTT CAGAGAGGTT 720
CTCTGTGTTG AGACATAAAT TTTGTAAAGG AGATTATGGA GAGGAGAAGC AAGTTGTTGA 780
AGATTAAGTA AAGAAGGTGG ACAAATCTAT TTTGTCAGAA ATTCAGGCCA AGGGGGAGAT 840
TTGTTGGGTT TTATTTGCCC TGATTTTTTA CCATAAATAG GTTTTCCTTT AAGGAAAAGG 900
TTTTGAATTG ACTATTCTTT TTTTGGTAGG AAAAGGTTTA GGATTCTATA AATAGAGGCA 960
TGTTCCTTCT AACTTAATTA GCATTCACAA TGTAGTTTTA AGGGCTTTGA GAGTTTTGGT 1020
TAGAGGGAGA ATTTGTGAAC CTCTCATGTA TTCCGAGTGA ATTGGTTGAG GTTGTTTCCC 1080
TCTGTATTTT GTACTCTCAT GTTTATAGTG GATTGCTCAT TTCCTTTGTG GACGTAGGTC 1140
GATTGACCGA ACCACGTTAA ATCTTTGTGT CTTTTGGTAT ATTTCTCGTT GTCTTCTTAC 1200
TCGTGGTCTT TCGAGGTTTG CTTTGCTAGC TTCCGCGTTT ACACCTGCTT ATTTGCGGTC 1260
CTAACAGAGT TCGATGGGTT GAATCTATAA AAAGAAAAAT ATACTCGTGA TTCACGATTA 1320
TTTATATGAA AATATAATAA ATATTGAATT TCCTTTGCTA TTTCTTATGT TTACGTCTTT 1380
ATATTTCAAA TTATTCCACC AATACTGACA AGCCCTAGGC CATCTCTAGG AAATTCATAC 1440
AATTTTTTTT TTGTTGTTAA CTAGTTAAAT TGGCAGCCTT AAAGATTATT GTAAAATTCA 1500
AGGCAACTTC CTCAAGTACT ACAACTACAT TGTAACATCC CAGTCAAAGT GTCCTAAAAT 1560
TTTATAAAAT TTGACACATG AAACAATAGC ACAATAAATT TTAGTACTAT TGCAGCCATG 1620
GCCCATAAGC CATCATGTAT TATAGTCAAA ATGGGTCCTT TTCCAATTTG TCTTGATCCC 1680
AAAATCCCTT TGTAGGTAAG ATGGTTCAAC AAGGAACTAT GACTCTTAAG GTAGACTTGG 1740
ACTCATAGAC TTGTCATAAC TCATAAAGAC TTGGAATATA ATAATTATTC ATTTAAATTA 1800
TAATTCTCTA CTTTAATATC TTCTACTATA AATACCCTTT CAAAGCCTCA TTATTTGTAC 1860
ATCAAACATT GATATTCATC TCTTCAATCT TTTGTATTCA CATATTCTAT TTATTCAATA 1920
CACTTAGGAA AACACTTTAC CAAGAAATTA AGATGGAGAA CTTCCCAATT ATTAACTTGG 1980
AAAAGCTCAA TGGAGATGAG AGAGCCAACA CCATGGAAAT GATCAAAGAT GCTTGTGAGA 2040
ATTGGGGCTT CTTTGAGGTA ATCATAAATT ACATAAACAT ATTAATATGT TTGTTTCAAT 2100
TTATCAGTCA TACTTTTCTC TGTTTTAAAA TTAATGTCAC TTTCAATATT TAATAATTCG 2160
CATGACATGT TTATAACACA ACAAGATATA GGTTACATTT TGATACATTA TATATAACTT 2220
CTGTCACACG ACTCAAAAGT CTTTCTTAAT TTCTTGAATT CAATGATCGA TCAAACTAAG 2280
ACACGTAAAA TGAAACGGGG AATAGTAATT CTGTTTGCTT ATGTGATCAT TGTAGTTGGT 2340
GAACCATGGA ATTCCACATG AAGTAATGGA CACAGTAGAG AAAATGACAA AGGGACATTA 2400

CAAGAAGTGC ATGGAACAGA GGTTTAAGGA ACTAGTGGCA AGTAAGGGAC TTGAGGCTGT 2460

TCAAGCTGAG GTTACTGATT TAGATTGGGA AAGCACTTTC TTCTTGCGCC ATCTTCCTAC 2520

TTCTAATATC TCTCAAGTAC CCGATCTTGA CGAAGAATAC AGGTACATAC ATGTGTCCTA 2580

CATATTGCGT ATATAATAAA TAAACACAAA ATTTAAGTTA TATACGCTGA CAGTATAACT 2640

AATTATAATG TTGTACCAAA TGATGCAGAG AGGTGATGAG AGATTTTGCT AAAAGATTGG 2700

AGAAATTGGC TGAGGAGTTA CTTGACTTAC TCTGTGAAAA TCTTGGACTT GAAAAAGGTT 2760

ACTTGAAAAA TGCCTTTTAT GGATCAAAAG GTCCCAACTT TGGTACTAAA GTTAGCAACT 2820

ATCCACCATG TCCTAAGCCC GATTTGATCA AGGGACTCCG CGCTCATACA GACGCAGGAG 2880

GCATCATACT TCTGTTCCAA GATGACAAAG TGAGTGGCCT TCAACTCCTC AAAGACGAGC 2940

AATGGATCGA TGTTCCTCCC ATGCGCCACT CTATTGTGGT TAACCTTGGT GACCAACTTG 3000

AGGTACAAGA TTCACTAAGT GTGTGTGTTT TTATCACTAT AACTTAGAAG TAGTAACTAA 3060

AAATGGTATT AATGAAATGT TATAAAAACA GGTGATCACT AACGGGAAGT ACAAGAGTGT 3120

GCTGCACAGA GTAATTGCAC AAACAGACGG GACACGAATG TCATTAGCCT CATTTTACAA 3180

TCCAGGAAGT GATGCAGTAA TATATCCAGC AAAAACTTTG GTTGAAAAAG AGGCAGAGGA 3240

AAGTACACAA GTGTATCCAA AGTTTGTGTT TGATGATTAC ATGAAGTTAT ATGCTGGACT 3300

CAAGTTTCAA GCCAAAGAGC CAAGATTTGA AGCAATGAAG GCAATGGAAA GTGATCCAAT 3360

TGCAAGTGCT TAGATCCCAA TTCAATTAAA AAAATTGGTG TTTGAAAAAT ATATTTAAAT 3420

ATAGCAATCT ATGTATACAC ATTATTTGCT CTTCTTATGT ATGGTAGAAT AAAGTTAGTA 3480

TTAAAAAAGA TTGTGATTTG CTGCATATGT ATCAAAAAGA GTCCTAATAT TTGTATCTAT 3540

AAATAAGGTG CCTTCTAGTG AAATTATACA AATAATAATT TGGAGTGTAT TGTTCTTTCT 3600

CATGTAATTT AACTTTTAAG TATCTTACTT TACAATATAC TGTTCACTTA TTGAACATAT 3660

TGAGTGATAT ATTGACTCAA T 3681

(2) INFORMATION FOR SEQ ID NO: 2:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 2

(xi) SEQUENCE DESCRIPTION: SEQ ID NO: 2:

CATTCATCTC TTCAATCTTT TG 22

(2) INFORMATION FOR SEQ ID NO: 3:

(i) SEQUENCE CHARACTERISTICS:
(A) LENGTH: 26 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 3

(xi) SEQUENCE DESCRIPTION: SEQ ID NO: 3:

CTTAATTTCT TGGTAAAGTG TTTTCC 26

(2) INFORMATION FOR SEQ ID NO: 4:

(i) SEQUENCE CHARACTERISTICS:
(A) LENGTH: 33 base pairs
(B) TYPE: nucleic acid
(C) STRANDEDNESS: unknown
(D) TOPOLOGY: unknown

(ii) MOLECULE TYPE: other nucliec acid
(A) DESCRIPTION: /desc = "PCR PRIMER"

(vii) IMMEDIATE SOURCE:
(B) CLONE: PCR PRIMER SEQ ID NO 4

(xi) SEQUENCE DESCRIPTION: SEQ ID NO: 4:

ATGGAGAACT TCCCAATTAT TAACTTGGAA AAG 33

(2) INFORMATION FOR SEQ ID NO: 5:

(i) SEQUENCE CHARACTERISTICS:
(A) LENGTH: 30 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 5

(xi) SEQUENCE DESCRIPTION: SEQ ID NO: 5:

CTAAGCACTT GCAATTGGAT CACTTTCCAT 30

(2) INFORMATION FOR SEQ ID NO: 6:

(i) SEQUENCE CHARACTERISTICS:
(A) LENGTH: 32 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 6

(xi) SEQUENCE DESCRIPTION: SEQ ID NO: 6:

CTTTACCAAG AAGTGCACAT GGAGAACTTC CC 32

(2) INFORMATION FOR SEQ ID NO: 7:

(i) SEQUENCE CHARACTERISTICS:
(A) LENGTH: 28 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 7

(xi) SEQUENCE DESCRIPTION: SEQ ID NO: 7:

GAATTGGGCC CTAAGCACTT GCAATTGG 28

(2) INFORMATION FOR SEQ ID NO: 8:

(i) SEQUENCE CHARACTERISTICS:
(A) LENGTH: 25 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 8

(xi) SEQUENCE DESCRIPTION: SEQ ID NO: 8:

GCTGGACTCA AGTTTCAAGC CAAAG 25

(2) INFORMATION FOR SEQ ID NO: 9:

(i) SEQUENCE CHARACTERISTICS:
(A) LENGTH: 23 base pairs

(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 9

(xi) SEQUENCE DESCRIPTION: SEQ ID NO: 9:

CCATCTCATA AATAACGTCA TGC 23

(2) INFORMATION FOR SEQ ID NO: 10:

(i) SEQUENCE CHARACTERISTICS:
(A) LENGTH: 1949 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA"

(vii) IMMEDIATE SOURCE:
(B) CLONE: PHIR-ACO SEQ ID NO 10

(xi) SEQUENCE DESCRIPTION: SEQ ID NO: 10:

ACCGGTGAAT TCCCATGGAG TCAAAGATTC AAATAGAGGA CCTAACAGAA CTCGCCGTAA 60

AGACTGGCGA ACAGTTCATA CAGAGTCTCT TACGACTCAA TGACAAGAAG AAAATCTTCG 120

TCAACATGGT GGAGCACGAC ACGCTTGTCT ACTCCAAAAA TATCAAAGAT ACAGTCTCAG 180

AAGACCAAAG GGCAATTGAG ACTTTTCAAC AAAGGGTAAT ATCCGGAAAC CTCCTCGGAT 240

TCCATTGCCC AGCTATCTGT CACTTTATTG TGAAGATAGT GGAAAAGGAG GTGGCTCCTA 300

CAAATGCCAT CATTGCGATA AAGGAAAGGC CATCGTTGAA GATGCCTCTG CCGACAGTGG 360

TCCCAAAGAT GGACCCCCAC CCACGAGGAG CATCGTGGAA AAAGAAGACG TTCCAACCAC 420

GTCTTCAAAG CAAGTGGATT GATGTGATAT CTCCACTGAC GTAAGGGATG ACGCACAATC 480

CCACTATCCT TCGCAAGACC CTTCCTCTAT ATAAGGAAGT TCATTTCATT TGGAGAGGAC 540

AGGGTACCGC GGCACGGCCA GCCACGCCGC TGAGCCCGCA GTTTCTCGAG TTTCTGCGGG 600

CTCAGCGGCG TGGCTGGCCG TGCCGCCCAT GGGCGGCGGG GCTGCAGGAA TTCGATATCA 660

AGCTTATCGA TACCGTCGAC CTCGAGGGGG GGCCCGGTAC CGGATCCCCT GCACATGGAG 720

AACTTCCCAA TTATTAACTT GGAAAAGCTC AATGGAGATG AGAGAGCCAA CACCATGGAA 780

ATGATCAAAG ATGCTTGTGA GAATTGGGGC TTCTTTGAGT TGGTGAACCA TGGAATTCCA 840

CATGAAGTAA TGGACACAGT AGAGAAAATG ACAAAGGGAC ATTACAAGAA GTGCATGGAA 900

CAGAGGTTTA AGGAACTAGT GGCAAGTAAG GGACTTGAGG CTGTTCAAGC TGAGGTTACT 960

GATTTAGATT GGGAAAGCAC TTTCTTCTTG CGCCATCTTC CTACTTCTAA TATCTCTCAA 1020

GTACCCGATC TTGACGAAGA ATACAGAGAG GTGATGAGAG ATTTTGCTAA AAGATTGGAG 1080

AAATTGGCTG AGGAGTTACT TGACTTACTC TGTGAAAATC TTGGACTTGA AAAAGGTTAC 1140

TTGAAAAATG CCTTTTATGG ATCAAAAGGT CCCAACTTTG GTACTAAAGT TAGCAACTAT 1200

CCACCATGTC CTAAGCCCGA TTTGATCAAG GGACTCCGCG CTCATACAGA CGCAGGAGGC 1260

ATCATACTTC TGTTCCAAGA TGACAAAGTG AGTGGCCTTC AACTCCTCAA AGACGAGCAA 1320

TGGATCGATG TTCCTCCCAT GCGCCACTCT ATTGTGGTTA ACCTTGGTGA CCAACTTGAG 1380

GTGATCACTA ACGGGAAGTA CAAGAGTGTG CTGCACAGAG TAATTGCACA AACAGACGGG 1440

ACACGAATGT CATTAGCCTC ATTTTACAAT CCAGGAAGTG ATGCAGTAAT ATATCCAGCA 1500

AAAACTTTGG TTGAAAAAGA GGCAGAGGAA AGTACACAAG TGTATCCAAA GTTTGTGTTT 1560

GATGATTACA TGAAGTTATA TGCTGGACTC AAGTTTCAAG CCAAAGAGCC AAGATTTGAA 1620

GCAATGAAGG CAATGGAAAG TGATCCAATT GCAAGTGCTT AGGGGAGCCT GGGCCCCTGC 1680

AGGTCGTTCA AACATTTGGC AATAAAGTTT CTTAAGATTG AATCCTGTTG CCGGTCTTGC 1740

GATGATTATC ATATAATTTC TGTTGAATTA CGAATTGCAT GTAATAATTA ACATGTAATG 1800

CATGACGTTA TTTATGAGAT GGGTTTTTAT GATTAGAGTC CCGCAATTAT ACATTTAATA 1860

CGCGATAGAA AACAAAATAT AGCGCGCAAA CTACCATAAA TTATCGCGCG CGGTGTCATC 1920

TATGTTACTA GATCGGGAAG CTTACCGGT 1949

(2) INFORMATION FOR SEQ ID NO: 11:

(i) SEQUENCE CHARACTERISTICS:
(A) LENGTH: 78 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: SYNTHERTIC INVERTED REPEAT SEQ ID NO 11

(xi) SEQUENCE DESCRIPTION: SEQ ID NO: 11:

GCGGCACGGC CAGCCACGCC GCTGAGCCCG CAGTTTCTCG AGTTTCTGCG GGCTCAGCGG 60

CGTGGCTGGC CGTGCCGC 78

(2) INFORMATION FOR SEQ ID NO: 12:

(i) SEQUENCE CHARACTERISTICS:
(A) LENGTH: 75 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PRIMER"

(vii) IMMEDIATE SOURCE:
(B) CLONE: PCR PRIMER SEQ ID NO 12

(xi) SEQUENCE DESCRIPTION: SEQ ID NO: 12:

GCGGGTACCG CGGCACGGCC AGCCACGCCG CTGAGCCCGC AGTTTCTCGA GGATGGGTTG 60

GCTCCATGGG CGGCG 75

(2) INFORMATION FOR SEQ ID NO: 13:

(i) SEQUENCE CHARACTERISTICS:
(A) LENGTH: 75 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 13

(xi) SEQUENCE DESCRIPTION: SEQ ID NO: 13:

CGCCGCCCAT GGAGCCAACC CATCCTCGAG AAACTGCGGG CTCAGCGGCG TGGCTGGCCG 60

TGCCGCGGTA CCCGC 75

(2) INFORMATION FOR SEQ ID NO: 14:

(i) SEQUENCE CHARACTERISTICS:
(A) LENGTH: 66 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 14

(xi) SEQUENCE DESCRIPTION: SEQ ID NO: 14:

GGGGCGCCGC TCGAGTTTCT GCGGGCTCAG CGGCGTGGCT GGCCGTGCCG CCCATGGCGC 60

ATCGGG 66

(2) INFORMATION FOR SEQ ID NO: 15:

(i) SEQUENCE CHARACTERISTICS:
(A) LENGTH: 66 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "PCR PRIMER"

(vii) IMMEDIATE SOURCE:
(B) CLONE: PCR PRIMER SEQ ID NO 15

(xi) SEQUENCE DESCRIPTION: SEQ ID NO: 15:

CCCTAGGCGC CATGGGCGGC ACGGCCAGCC ACGCCGCTGA GCCCGCAGAA ACTCGAGCGG 60

CGCCCC 66